Europäisches Patentamt

European Patent Office

Office européen des brevets

(19)

(11) Publication number: **0 032 825**
A2

(12)

# EUROPEAN PATENT APPLICATION

(21) Application number: **81300198.9**

(22) Date of filing: **16.01.81**

(51) Int. Cl.³: **A 61 K 9/08**, A 61 K 31/225

(30) Priority: **18.01.80 US 113154**

(43) Date of publication of application: **29.07.81**
**Bulletin 81/30**

(84) Designated Contracting States: **CH DE FR GB LI NL SE**

(71) Applicant: **MALLINCKRODT, INC., 675 Brown Road P.O. Box 5840, St. Louis, MO 63134 (US)**

(72) Inventor: **Piper, Roger, 908 Oge Avenue, DesPeres Missouri 63101 (US)**

(74) Representative: **Eyles, Christopher Thomas et al, BATCHELLOR, KIRK & EYLES 2 Pear Tree Court Farringdon Road, London, EC1R 0DS (GB)**

(54) Stabilized solutions of dioctyl calcium sulfosuccinate in corn oil.

(57) A solution of dioctyl calcium sulfosuccinate in corn oil includes, as a gel retarding agent, an amount of about 1 to about 5 percent by volume of a pharmaceutically acceptable low molecular weight alkanol.

EP 0 032 825 A2

ACTORUM AG

STABILIZED SOLUTIONS OF DIOCTYL CALCIUM
SULFOSUCCINATE IN CORN OIL.

This invention relates to stabilized solutions of
dioctyl calcium sulfosuccinate in corn oil.

Dioctyl calcium sulfosuccinate is sold as a stool
softener laxative in the form of soft gelatin capsules. It
is preferred over dioctyl sodium sulfosuccinate for people
with restricted sodium intake. Commercially, the dioctyl
calcium sulfosuccinate is dissolved in a non-aqueous
solvent such as corn oil to which a small amount of
glycerol monooleate is added, ostensibly to prevent
gelling, see U.S. Patent No. 3,035,973.

The problem with such commercially available
formulations is that the solution does gel within a
relatively short time. This presents no problem if the
material has already been encapsulated but results in waste
if it has not yet been injected into the capsules.

In view of the above, among the objects of the
present invention may be noted the provision of a solution
of dioctyl calcium sulfosuccinate in corn oil which is
stabilized against gelling for a relatively long time, for
example, for three months. Other objects and features will
be in part apparent and part pointed out hereinafter.

The invention accordingly comprises the solutions,
dosage units, capsules, and methods for production of the
solutions hereinafter described, the scope of the invention
being indicated by the subjoined claims.

The stabilized solutions of the present invention
essentially include a gel retarding amount of a low
molecular weight alkanol such as ethyl alcohol or
isopropyl alcohol. Since the product solutions are
intended for oral ingestion, the alcohol must of necessity

— 2 —

0032825

be pharmaceutically acceptable, like ethyl alcohol, for
such use. Where permitted, other alkanols such as
isopropyl alcohol, alone or in admixture, may be used.

In a preferred embodiment of the invention, in a
solution otherwise corresponding to commercially available
products, a suitable dosage unit includes from about 50 to
about 300mg of dioctyl calcium sulfosuccinate and,
optionally, from about 1 to about 10 percent by volume of
glycerol monooleate in sufficient corn oil to make a
solution. In this is incorporated, in accordance with the
present invention, from about 1 to about 5 percent by
volume of a low molecular weight alkanol such as ethyl
alcohol, isopropyl alcohol, or a mixture thereof. To
ensure against gelling for about three months, it is
preferred if ethyl alcohol be present in an amount of from
about 2 to about 4 percent, e.g. about 2 percent to about 3
percent, by volume.

For some purposes, the glycerol monooleate may be
omitted, in which case higher amounts of alkanol are
required. For example, to insure against gelling in the
absence of glycerol monooleate for as much as three months,
it is preferred that at least about 3 percent by volume of
ethyl alcohol be included.

The solutions of the present invention are easily
prepared by predissolving the dioctyl calcium
sulfosuccinate in the alkanol, with or without the
application of heat. To this solution is added the corn
oil and the glycerol monooleate, if any. Since an excess
amount of alkanol is needed to solubilize the dioctyl
calcium sulfosuccinate, and since excess amounts of alkanol
are to be avoided if the stabilized suspension is to be
used in soft shelled gelatin capsules, excess amounts of
alkanol are removed by distillation or by evaporation at normal pressure or under vacuum.

The following examples illustrate the invention.

## Example 1

Dioctyl calcium sulfosuccinate is prepared by converting the more commonly available dioctyl sodium sulfosuccinate into dioctyl calcium sulfosuccinate by acidifying a solution of dioctyl sodium sulfosuccinate in isopropyl alcohol with sulfuric acid to precipitate out sodium sulfate. The reaction mixture is then neutralized with calcium hydroxide to form the calcium salt of the dioctyl ester of sulfosuccinate.

## Example 2

A solution of dioctyl calcium sulfosuccinate as described in Example 1 was evaporated to near dryness on a steam bath and then dried in a vacuum oven at 80° for 2 hours. A 21g sample of the solid was dissolved in a solution containing 18g of corn oil and 2g of glycerol monooleate by stirring and heating to 70-75°C for one and one-half hours. The product of this process is like that described in Patent No. 3,035,973 and is not an embodiment of the present invention. The stability of this solution along with the other solutions prepared in the following examples is described in Table 1.

## Example 3

A solution of dioctyl calcium sulfosuccinate dissolved in isopropyl alcohol containing 50g of dioctyl calcium sulfosuccinate was mixed with 45g of corn oil and 5g of glycerol monooleate. Most of the isopropyl alcohol was removed by slow heating under vacuum. A small amount of isopropyl alcohol, as determined by gas chromotography, was left in the solution.

## Example 4

The procedure of Example 3 was followed, except that the last traces of isopropyl alcohol were stripped from the solution by bubbling a stream of nitrogen through the solution for one hour at 90°C. This solution was then divided into three portions to which were added 0.4, 0.5 and 1.5 percent by volume of ethyl alcohol, respectively.

## Example 5

A solution of dioctyl calcium sulfosuccinate in isopropyl alcohol containing 156g of dioctyl calcium sulfosuccinate was mixed with 139g of corn oil and 15g of glycerol monooleate, and heated under vacuum to 130°C to remove almost all of the isopropyl alcohol. Portions of this solution were mixed with almost 12% by volume of ethyl alcohol and part of the ethyl alcohol was then removed by vacuum distillation. The distillation of the ethyl alcohol removed the last traces of isopropyl alcohol by co-distillation. By stopping the distillation at 80-130°C, 0.5 to 2.1 percent by volume of ethyl alcohol was left in the solution.

## Example 6

Following the procedure of Example 5, a solution of dioctyl calcium sulfosuccinate in isopropyl alcohol was prepared containing 50g of dioctyl calcium sulfosuccinate mixed with 48g of corn oil with no glycerol monooleate. Ethyl alcohol was added to portions of this solution and then part of the ethyl alcohol removed by vacuum distillation.

- 5 -

## Table 1

| Example | Alcohol Type | Alcohol Concentration | Days Before Gelling |
|---------|--------------|----------------------|---------------------|
| 2 | | | 35 |
| 3 | isopropyl | 1.6% | 44 |
| 3 | isopropyl | 4.2% | 195 |
| 4 | ethyl | 0.4% | 19 |
| 4 | ethyl | 0.5% | 27 |
| 4 | ethyl | 1.5% | 62 |
| 5 | ethyl | 0.4% | 19 |
| 5 | ethyl | 1.0% | 42 |
| 5 | ethyl | 1.9% | 82 |
| 5 | ethyl | 2.1% | >105* |
| 5 | ethyl | 3.1% | >105* |
| 5 | ethyl | 5.1% | >105* |
| 6 | ethyl | 1.5% | 31 |
| 6 | ethyl | 3.0% | >105* |
| 6 | ethyl | 5.1% | >105* |

*Samples had not gelled after 105 days.

In view of the above, it will be seen that the several objects of the invention can be achieved and other advantageous results attained. As various changes could be made in the above compositions without departing from the scope of the invention, it is intended that all matter contained in the above description shall be interpreted as illustrative and not in a limiting sense.

CLAIMS.

1.      A solution of dioctyl calcium sulfosuccinate in corn oil including from about 1 to about 5 percent by volume of at least one pharmaceutically acceptable low molecular weight alkanol.

2.      A solution according to claim 1 wherein the alkanol is selected from the group consisting of ethyl alcohol, isopropyl alcohol and mixtures thereof.

3.      A solution according to claim 1 or claim 2 wherein the alkanol is ethyl alcohol.

4.      A solution according to claim 3 wherein the ethyl alcohol is present in an amount from about 2 to about 4 percent by volume.

5.      A solution according to any one of claims 1 to 4, additionally including glycerol monooleate present in an amount of from about 1 to about 10 percent by volume.

6.      A dosage unit comprising an amount of a solution according to any one of claims 1 to 5 which includes from about 50 to about 300mg of dioctyl calcium sulfosuccinate.

7.      Soft shelled gelatin capsules each containing a dosage unit according to claim 6.

8.      The use of a solution according to any one of claims 1 to 5, of a dosage unit according to claim 6, or of a capsule according to claim 7, as a stool softener laxative.

9.      A method of forming a solution of dioctyl calcium sulfosuccinate which comprises predissolving dioctyl calcium sulfosuccinate in a pharmaceutically acceptable low molecular weight alkanol, adding corn oil thereto, and removing sufficient excess alkanol to provide a solution containing dioctyl calcium sulfosuccinate in corn oil including from about 1 to about 5 percent by volume of the alkanol.

10.     A method according to claim 9 wherein, in addition to corn oil, there is added sufficient glycerol monooleate to provide from about 1 to about 10 percent by volume thereof in the solution following removal of the excess alkanol.